# EUROPEAN PATENT APPLICATION

(11) **EP 1 483 957 A1**
(43) Date of publication of application: **08.12.2004**
(21) Application number: 04102500.8
(22) Date of filing: 03.06.2004
(51) Int. Cl.: A01G 5/00, A41G 1/00

(54) **Bouquet of cut flowers or plants and a device provided with a reservoir containing a flavouring substance**

(30) Priority: 03.06.2003 NL 1023589
(71) Applicant: Koos Lamboo Dried Flowers Vof, 2161 CK Lisse (NL)
(72) Inventor: Lamboo, Koos, 2161 CK, Lisse (NL)
(74) Representative: Beetz, Tom

(57) **Abstract**

The invention relates to a bouquet of cut flowers (1) and/or plants and a device (2) provided with a reservoir containing a flavoring substance for releasing the flavoring substance to the surroundings, characterized in that the reservoir comprises a material containing pores wherein a solution or suspension of the flavoring substance is absorbed. The invention further relates to a flavoring substance releasing device (2) having the shape of a stem (3) of which at least the kernel (4) is a material containing pores that serves as reservoir to a therein absorbed solution or suspension of a flavoring substance, wherein the kernel (4) optionally is at least partially surrounded by an outer wall, that optionally is at least partially pervious to the solution or suspension of the flavoring substance, and also to a method for manufacturing this device.

## Description

The present invention relates to a bouquet of cut flowers and/or plants and a device provided with a reservoir containing a flavoring substance, to a device releasing flavoring substance, and also to a method of manufacture of said device.

Particularly, as a result of breeding and selection of flowers and plants for obtaining a production that is as large as possible and for obtaining resistance against diseases, the natural property of flowers and plants to release a scent is weakened substantially. Many commercial cut flowers and plants nowadays do not or virtually do not release any scent, or even release an unpleasant scent. However, to the consumer it is of importance that flowers and plants smell agreeable. In the past some attempts have been made to find a solution to this problem. None of these solutions, however, has lead to a commercial success.

According to Dutch patent application NL 1000820 cut flowers were provided with a flavoring substance by means of a method wherein a flavoring substance was brought onto cut flowers, by treating these with a mixture of a flavoring substance, a solvent, and a binder. However, in practice it appeared that most flowers did not stand such treatment because the pores of the flower leaves were clogged by this treatment, leading to a faster dying of the flowers.

In French patent application FR 2,637,167 a bouquet of flowers has been disclosed which contain a device for releasing a scent. The device has large dimension in comparison with the bouquet and contains an electrically driven diffuser to release droplets of ethereal oils or perfumes. In fact the flowers, usually artificial flowers, only serve to hide the device from view. This device is expensive, aesthetically almost unacceptable, has not for its object to provide cut flowers and the like with a scent, and must be refilled by the consumer himself.

The present invention has to its object to find a method and device wherein cut flowers and/or plants are provided with a scent, that has no negative influence on the life time of the flowers and/or plants and that moreover can be provided is a simple and cheap manner, without the need for electricity, which is esthetically acceptable, and in principle can be thrown away with the flowers or plants when died.

The inventors have succeeded in developing such a simple method to give a scent to a bouquet of flowers and/or plants. To this end the invention relates to a bouquet of cut flowers and/or plants and a device provided with a reservoir containing a flavoring substance for releasing the flavoring substance to the surroundings, characterized in that the reservoir comprises a material containing pores wherein a solution or suspension of the flavoring substance is absorbed.

The flavoring substance in principle can be any required flavoring substance, but preferably it is a flower or plant scent that fits to the bouquet. The term "bouquet" means at least one flower or plant and the device according to the invention. Usually the bouquet will contain a plurality of flowers or plants, and optionally other elements such as fern, leaves, branches, berries, and the like.

The reservoir comprises material containing pores, such as a sponge-like material wherein the pores have absorbed the flavoring substance, which is released thereof again to the surroundings.

There is further an aesthetic advantage if the device has a shape and size that are about those of the stems of the cut flowers or plants of the bouquet, so that the device is recognized as little as possible as a strange object in the bouquet. This can be obtained the best by using a stem as the device, particularly by using a reed stem or a reed stem-like element, of which the kernel serves as reservoir that optionally is at least partially surrounded by an outer wall that optionally at least is partially pervious to the solution or suspension of the flavoring substance. When the kernel is completely surrounded by an outer wall it is mandatory that the outer wall is at least partially pervious to the flavoring substance and it should remain possible to fill the reservoir through the outer wall with the flavoring substance that is dissolved or suspended in a solvent or suspension medium. It is therefore preferred to split the stem longitudinally, in order to bring the kernel partially to the surface, which makes both absorption and release of the flavoring substance simple. A very suitable stem is a reed stem that is known under the name hogla. Other types of reed stems of which the kernel is sponge-like can also be used.

One or a plurality of leave-shaped elements can be attached to the stem. This element can be as well a real flower leave or plant leave, as an artificial leave. The latter is preferred because usually this is easier to attach to the reed stem, for instance by gluing it thereto using common glue. Other methods of attachment are of course also possible, for instance by stapling the leave-shaped element to the reed stem. This leave-shaped element serves as well an aesthetic function to embellish the device and to give the bouquet as a whole an attractive appearance, as well as a technical advantage because this leave-shaped element increases the surface for evaporation of the flavoring substance when this leave-shaped element is in contact with the reservoir, and thereby the effectiveness to release the flavoring substance to the surroundings.

A stem, such as for instance a reed stem or a reed stem-like element, has not been used earlier as flavoring substance releasing device for this purpose. The invention therefore also relates to the flavoring substance releasing device having the shape of a stem of which at least the kernel is a material containing pores that serves as reservoir for a therein absorbed solution or suspension of a flavoring substance, wherein the kernel optionally is at least partially surrounded by an outer wall, that optionally is at least partially pervious to the solution or suspension of the flavoring substance. If required, to this device one or a plurality of leave-shaped elements can be attached, as has been described hereinabove. To obtain a fast take up of the flavoring substance-containing solution or suspension, it is preferred to use a stem that is split in the longitudinal direction. In that case the kernel becomes freely accessible to obtain a fast take up and favorable release of the flavoring substance.

In the figures an embodiment of a flavoring substance releasing device according to the invention has been shown schematically.

Fig. 1 shows a bouquet of cut flowers and a flavoring substance releasing device.

Fig. 2 shows a reed stem provided with a leave-shaped element.

Fig. 3 shows a detailed view of a part of the reed stem according to Figure 2 at a larger scale.

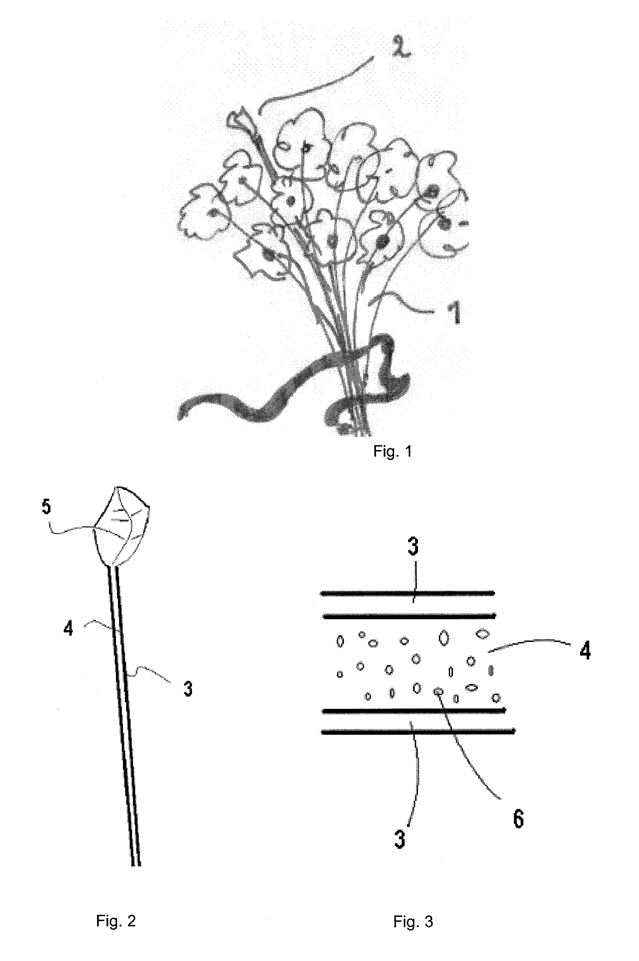

In Figure 1 an embodiment has been given of a bouquet of cut flowers 1 and a flavoring substance releasing device 2, wherein the device has the shape of a stem.

In Figure 2 an embodiment of a flavoring substance releasing device 2 has been shown that has the shape of a reed stem which is shown here with an outer wall 3, a kernel 4 that serves as reservoir and a leave-shaped element 5. The leave shaped element 5, for instance, is glued to the outer wall 3 of the reed stem. The kernel 4 is visible because the reed stem is split in the longitudinal direction. To improve the appearance of the device further, and thereby to increase acceptation of the device in the bouquet by the consumer, the leave shaped elements 5 is attached to the reed stem 2. This also increases the available surface for evaporation of the flavoring substance. If required, of course more leave shaped elements 5 can be attached to the device.

In figure 3 a detailed view of this reed stem is shown with the outer wall 3, the kernel 4 and pores 6 that are present in the kernel 4. This kernel 4 of pores 5 form together an absorption element that serves as a reservoir for the solution or suspension of flavoring substance. If the reed stem is not split this detailed view corresponds with a longitudinal section of the stem.

The invention further relates to a method for the manufacture of the herein mentioned device, in which a stem, in particular a reed stem or a reed stem-like element, of which the kernel consists of a material containing pores, is brought in contact with a solution or suspension of a flavoring substance for a sufficient time for the kernel to absorb the required amount of the flavoring substance. The contact of the kernel and the solution or suspension can, for instance, be brought about by immersing, injecting, impregnating, or spraying the device. In this manner the (pore-containing sponge-like) interior of a longitudinally split hogla reed stem can be completely saturated with a flavoring substance-containing solution or suspension within 24 hours by immersing. When saturated with flavoring substance, the flavoring substance can be released again slowly by evaporation from the reservoir. It is preferred that this evaporation process at least continues as long as the lifetime of the cut flowers. Preferably, this is at least a week. It is also possible that the flavoring substance is slowly released to the water from the part of the device that together with the bouquet is in contact with the water, and is evaporated from the water to release its scent.

The flavoring substance can be a usual flavoring substance, particularly a flower scent. These flavoring substances contain ethereal oils, perfumes, perfume oils, and the like. Examples of flavoring substances are Rose Sonia, Rose Tros, freesia, carnation, lily-of-the-valley, lilac, floral bouquet, sweet freesia, Rose Fruity, Banana sweet, pine aroma, menthol, and cinnamon.

The solution or suspension can be aqueous or organic, or a mixture thereof. Organic solvents are for instance alcohols, particularly mono and poly alcohols such as propanol, glycol, ethylene glycol, and the like, or ethers such as polyethylene glycol, or thereof derived solvents such as monopropylene chloride. The solution or suspension can optionally contain other compounds, such as emulsifiers (for instance ethylene(20)-sorbitan monolaurate), preservation agents, feeding agents, surfactants such as Dow® 67 and/or agents to increase the lifetime of cut flowers.

## Claims

1. Bouquet of cut flowers and/or plants (1) and a device (2) provided with a reservoir containing a flavoring substance for releasing the flavoring substance to the surroundings, **characterized in that** the reservoir comprises a material containing pores (6) wherein a solution or suspension of the flavoring substance is absorbed.

2. Bouquet of claim 1, **characterized in that** the flavoring substance is a flavoring substance having a scent of flowers, plants, or trees.

3. Bouquet according claim 1 or 2, **characterized in that** the device (2) has a shape and size that are about those of the stems of the cut flowers in the bouquet.

4. Bouquet of claim 3, **characterized in that** the device (2) is a stem of which the kernel (4) serves as reservoir and optionally is at least partially surrounded by an outer wall (3), that optionally is at least partially pervious to the solution or suspension of the flavoring substance.

5. Bouquet of claim 4, **characterized in that** the stem is split longitudinally.

6. Bouquet of claim 4 or 5, **characterized in that** the stem is a reed stem of the type hogla.

7. Bouquet according to any one of claims 4-6, **characterized in that** the stem is provided with a leave-shaped element (5).

8. Flavoring substance releasing device (2) having the shape of a stem of which at least the kernel (4) is a material containing pores (6) that serves as reservoir to a therein absorbed solution or suspension of a flavoring substance, wherein the kernel optionally is at least partially surrounded by an outer wall (3), that optionally is at least partially pervious to the solution or suspension of the flavoring substance.

9. Device of claim 8, **characterized in that** at least a leave-shaped element (5) is attached to the device (2).

10. Device of claim 9 or 10, **characterized in that** the stem is split in the longitudinal direction.

11. Method for the manufacture of the device according to any one of claims 8 - 10 in which a stem of which the kernel is a material containing pores, is brought in contact to a solution or suspension of a flavoring substance for a sufficient time for the kernel to absorb the required amount of the flavoring substance.
